# EUROPÄISCHE PATENTSCHRIFT

(11) **EP 2 292 194 B1**
(45) Veröffentlichungstag und Bekanntmachung des Hinweises auf die Patenterteilung: **23.09.2015**
(21) Anmeldenummer: 10012107.8
(22) Anmeldetag: 16.11.2004
(51) Int. Cl.: A61F 9/009, A61F 9/01

(54) **Adapter zum mechanischen Koppeln einer Laserbearbeitungsvorrichtung mit einem Objekt**
Adapter for mechanically coupling a laser processing device to an object
Adaptateur destiné au couplage mécanique d'un dispositif d'usinage au laser et d'un objet

(30) Priorität: 19.11.2003 DE 10354025
(43) Veröffentlichungstag der Anmeldung: 09.03.2011
(62) Teilanmeldung aus: 04797937.2
(73) Patentinhaber: Carl Zeiss Meditec AG, 07745 Jena (DE)
(72) Erfinder: Mühlhoff, Dirk, 07751 Jena (DE); Ebert, Elke, 07743 Jena (DE); Festag, Karsten, 07749 Jena (DE); Wolf, Uwe, 99441 Magdala (DE)
(74) Vertreter: Geyer, Fehners & Partner

(56) Entgegenhaltungen:
- EP-A- 0 336 065
- WO-A1-03/002008
- DE-A1- 19 831 674
- US-A- 4 718 418
- US-A1- 2001 021 844

## Beschreibung

Die Erfindung bezieht sich auf einen Adapter zum mechanischen Koppeln einer Laserbearbeitungsvorrichtung mit einem Objekt, der aufweist einen zentralen Bereich, welcher in den auf einer optischen Achse zum Objekt verlaufenden Strahlengang der Laserbearbeitungsvorrichtung schaltbar ist, und einen außerhalb des zentralen Bereichs liegenden Randbereich, wobei der Adapter einerseits an der Laserbearbeitungsvorrichtung befestigbar und andererseits mit seinem zentralen Bereich zur Anlage am Objekt ausgebildet ist.

Bei der Materialbearbeitung wird oft eine Laserbearbeitungsvorrichtung zum Abrastern der zu bearbeitenden Gebiete des Objektes mit einem Laserstrahl eingesetzt. Die Genauigkeit der Positionierung des Laserstrahls bestimmt dabei in der Regel die bei der Bearbeitung erzielte Präzision. Wird der Laserstrahl in ein Bearbeitungsvolumen fokussiert, bedarf es einer exakten dreidimensionalen Positionierung. Für eine hochgenaue Bearbeitung ist es deshalb in der Regel unerlässlich, das Objekt in exakt definierter Lage zur Laserbearbeitungsvorrichtung zu halten. Für solche Anwendungen dient der eingangs genannte Adapter, da mit ihm das zu bearbeitende Objekt fixiert werden kann, wodurch definierte Verhältnisse bis zum Bearbeitungsvolumen erreichbar sind. Der zentrale Bereich des Adapters wird damit Teil des Strahlenganges.

Dies ist insbesondere bei der Mikrobearbeitung von Materialien notwendig, die nur eine geringe lineare optische Absorption im Spektralbereich der bearbeitenden Laserstrahlung aufweisen. Bei solchen Materialien werden üblicherweise nicht-lineare Wechselwirkungen zwischen Laserstrahlung und Material ausgenutzt, meist in Form eines optischen Durchbruches, der im Fokus des Laserstrahls erzeugt wird. Da die bearbeitende Wirkung dann nur im Laserstrahlfokus stattfindet, ist es unerlässlich, den Fokuspunkt exakt dreidimensional auszurichten. Zusätzlich zu einer zweidimensionalen Ablenkung des Laserstrahls ist somit eine exakte Tiefeneinstellung der Fokuslage im Strahlengang erforderlich. Der eingangs genannte Adapter dient dazu, konstante und auch mit einer gewissen Genauigkeit bekannte optische Verhältnisse im Strahlengang zum Objekt sicherzustellen, indem sein zentraler Bereich Teil des Strahlenganges ist und er Objekt und Laserbearbeitungsvorrichtung koppelt.

Eine typische Anwendung für einen solchen Adapter ist das als Femtosekunden-LASIK bekannte augenoptische Operationsverfahren, bei dem die als Therapiegerät ausgebildete Laserbearbeitungsvorrichtung einen Laserstrahl auf einen Fokuspunkt in der Größenordnung eines Mikrometers in die Hornhaut fokussiert. Im Fokus entsteht dann ein Plasma, das eine lokale Trennung des Hornhautgewebes bewirkt. Durch geeignete Aneinanderreihung der auf diese Weise erzeugten lokalen Trennungszonen werden mikroskopische Schnitte realisiert und ein bestimmtes Hornhautteilvolumen isoliert. Durch Entnahme des Teilvolumens wird dann eine gewünschte Brechungsänderung der Hornhaut erreicht, so dass eine Fehlsichtigkeitskorrektur möglich ist.

Für das LASIK-Verfahren ist aus der US 6373571 eine mit Referenzmarken versehene Kontaktlinse bekannt. Diese Kontaktlinse wird mittels einer separaten Messvorrichtung einjustiert, wodurch ein relativ aufwendiger Aufbau bedingt ist. Ein Beispiel für einen Adapter der genannten Art ist in der EP 1159986 A2 beschrieben. Er ähnelt der Kontaktlinse der US 6373571, weist aber zusätzlich einen Rand in Form einer Halterung mit Strichmarken auf, die dem Chirurgen eine visuelle Ausrichtung ermöglichen. Die US 2001/021884 A1 offenbart einen einstückigen Adapter.

Da der Adapter üblicherweise Kontakt mit dem zu bearbeitenden Objekt hat, ist es meist erforderlich, für jedes Objekt einen eigenen neuen Adapter einzusetzen. Dies gilt besonders unter dem Gesichtspunkt der Sterilität bei augenchirurgischen Verfahren. Daraus folgt, dass der Adapter jedes Mal vor einer Bearbeitung bzw. vor einem chirurgischen Eingriff an der Laserbearbeitungsvorrichtung, die dann als Therapiegerät ausgebildet ist, befestigt werden muss. Zur Befestigung ist es aus der WO 03/002008 A1 bekannt, das Kontaktglas in einer zangenartigen Einrichtung zu halten, die an der Laserbearbeitungsvorrichtung verriegelt ist. Die Verriegelung erfolgt über einen in einer Schiene geführten Kragen. Der Adapter wird quer zur optischen Achse formschlüssig eingeschoben.

In der DE 19831674 A1 ist die Verwendung eines mechanischen Kopplungsmechanismus beschrieben, bei dem ein in schrägem Winkel an einer Fassung eines Kontaktglases befestigter Metallstab mittels eines Magneten oder Elektromagneten in einer Hülse gehalten wird. Die Hülse sitzt ihrerseits an einem mechanischen Justiermechanismus, so dass die Stellung des Kontaktglases justiert werden kann.

Die Lösungen im Stand der Technik für das einen Adapter realisierende Kontaktglas benötigen zum einen durchwegs eine große Anzahl von komplex herzustellenden Bauteilen, zum anderen führen die im Stand der Technik verwendeten Befestigungen zu relativ groß bauenden Einheiten. Dies macht Kontaktgläsern des Standes der Technik für Vorrichtungen, die am Patienten ohne Vollanästhesie, sondern mit nur örtlicher Betäubung eingesetzt werden sollen, nachteilig. Nachteilig ist auch die große Menge der zur Herstellung dieses Einwegartikeis notwendigen Materials, insbesondere im Hinblick auf Wirtschaftlichkeit und Ökologie.

Der Erfindung liegt deshalb die Aufgabe zugrunde, einen Adapter der eingangs genannten Art baulich hinsichtlich der Befestigung an der Laserbearbeitungsvorrichtung zu vereinfachen, so dass insbesondere eine Anwendung auch ohne Vollnarkose möglich ist. Insbesondere soll eine Anwendung möglich sein, bei der der Adapter während bereits vollständig an der Laserbearbeitungsvorrichtung befestigt unter Lokalanästhesie am Patienten eingesetzt wird.

Die Erfindung ist im Anspruch 1 definiert.

Am Randbereich Befestigungsmittel vorgesehen sind, mit denen der Adapter lösbar an einer Aufnahme der Laserbearbeitungsvorrichtung insbesondere so befestigbar ist, dass bei Aufbringen einer bestimmten Zugkraft entlang der optischen Achse die Befestigungsmittel von der Aufnahme lösbar sind.

Die Aufgabe wird erfindungsgemäß gelöst mit einem Adapter der genannten Art, der durch eine axiale Bewegung (bezogen auf die optische Achse) an einer Aufnahme der Laserbearbeitungsvorrichtung ansetzt und lösbar ist, insbesondere mit einer Unterdruckbefestigung.

Die erfindungsgemäße Befestigung des Adapters an einer Aufnahme der Laserbearbeitungsvorrichtung erlaubt es nun, durch eine axiale Bewegung (bezogen auf die optische Achse der Laserbearbeitungsvorrichtung) den Adapter an der Laserbearbeitungsvorrichtung anzusetzen und auch wieder zu lösen. Dieses Vorgehen beruht auf der erstmals von den Erfindern gemachten und im Stand der Technik nicht angesprochenen Erkenntnis, dass es bei der Anwendung eines Adapters für ein augenchirurgisches Verfahren ohne Vollnarkose mitunter zu panikartigen Versuchen des Patienten kommen kann, seinen Kopf von der als Therapiegerät ausgebildeten Laserbearbeitungsvorrichtung wegzubewegen. Der erfindungsgemäße Adapter hat die vorteilhaften Eigenschaften eines "Panikverschlusses", der bei Wegziehen des Kopfes vom Therapiegerät aufgeht.

Der Adapter lässt sich dabei überraschend einfach dadurch realisieren, dass die Befestigungsmittel am Randbereich des Adapters vorgesehen werden, der beim Einsatz des Adapters nicht im Strahlengang der Laserbearbeitungsvorrichtung liegt.

Der erfindungsgemäße Adapter erlaubt eine einfach herzustellende Verbindung mit der Laserbearbeitungsvorrichtung, aufwendige Einsetz- und Justiervorgänge, wie sie im Stand der Technik unerlässlich sind, sind nicht nötig. Insbesondere erfolgt die Befestigung nicht zwingend in einer bestimmten Winkelausrichtung des Adapters, wie es beispielsweise bei der magnetisch arbeitenden Hülse der DE 19831674 A1 unerlässlich ist.

Der erfindungsgemäße Adapter ist darüber hinaus schnell und einfach wieder von der Laserbearbeitungsvorrichtung lösbar und öffnet insbesondere im Panikfall bei Überschreiten einer bestimmten Zugkraft entlang der optischen Achse. Schließlich erreicht der erfindungsgemäße Adapter den gewünschten platzsparenden Aufbau.

Die kompakte Bauweise, die für den erfindungsgemäßen Adapter möglich ist, erweist sich überraschenderweise auch aus psychologischen Gründen als Vorteil bei der Verwendung in augenchirurgischen Verfahren. Aufgrund des relativ kleinen, auf das Auge des Patienten aufzusetzenden Adapters empfinden Patienten den Eingriff nunmehr als deutlich weniger gefährdend oder bedrohend, wodurch das Auftreten von Paniksituationen, in denen die Panikverschlusseigenschaft des Adapters zum Tragen käme, deutlich vermindert ist.

Die Ausbildung der am Randbereich des Adapters ansetzenden Befestigungsmittel kann auf vielfältige Art und Weise im Rahmen der Erfindung erfolgen. So kommen elektrische oder pneumatische Wirkprinzipien gleichermaßen in Frage.

Im Falle der pneumatischen Befestigung ist eine Flanschfläche zur Vakuumbefestigung vorgesehen, und der Adapter wird in eine Bohrung eingelegt. Durch Anlegen eines Unterdruckes auf der mikroskopvorrichtungsseitig gelegenen Seite des Adapters, wird der Adapter in die Bohrung auf einen Anschlag gezogen und dort gehalten. Mit diesem Konzept ist zugleich eine gewisse Zentrierung des Adapters erreichbar Es ist deshalb bevorzugt, dass die Befestigungsmittel eine am Adapter vorgesehene Flanschfläche zur Vakuumbefestigung aufweisen. Dies ist ein Beispiel für eine Ausgestaltung, bei der die Befestigungsmittel den zentralen Bereich des Adapters zur optischen Achse der Laserbearbeitungsvorrichtung ausrichten. Dabei ist es besonders bevorzugt, die Flanschfläche so auszulegen, dass sie eine geringe Leckrate aufweist. Dadurch ist es möglich, die Flächen großzügig zu tolerieren und kostengünstig herzustellen. Die Vakuumpumpe ist in diesem Fall so dimensioniert, dass sie trotz bzw. bei gegebener Leckrate einen ausreichenden Unterdruck aufrechterhält.

Um die zentrierende Wirkung der Befestigung zu verstärken, ist der Randbereich des Adapters kegelstumpfförmig ausgebildet. Die Bohrung ist dann ebenfalls kegelförmig ausgelegt, so dass der kegelstumpfförmige Adapter vom Unterdruck in die sich verjüngende Bohrung gezogen wird. In einer alternativen Ausgestaltung dieses selbstzentrierenden Befestigungsmechanismus weitet sich der Randbereich des Adapters kegelförmig zur Laserbearbeitungsvorrichtung hin auf. Weist die Laserbearbeitungsvorrichtung eine dazu passende kegelstumpfförmige Aufnahme auf, wird auch dann der Adapter einfach mittels Unterdruck zentrierend an der Laserbearbeitungsvorrichtung befestigt.

Die bezüglich Unterdruckbefestigung geschilderten Ansätze können natürlich auch bei Verwendung entsprechender Materialien unter Ausnutzung elektromagnetischer oder -statischer Effekte verwendet werden.

Zur Verbesserung der optischen Eigenschaften des Adapters ist es ggf. sinnvoll, flüssige Kontaktmittel an dessen optischen Grenzflächen einzusetzen. Dieses in der Mikroskopie bekannte Prinzip der Immersionsoptiken verbessert die Haftungs- und optischen Eigenschaften des Adapters.

Ein Anwendungsgebiet für den erfindungsgemäßen Adapter, bei dem dessen Vorteile besonders zur Geltung kommen ist, wie bereits erwähnt, die Augenchirurgie. Es ist deshalb bevorzugt, dass der zentrale Bereich des Adapters als Kontaktglas zum Auflegen auf das Objekt, insbesondere auf die Augenhornhaut, ausgebildet ist. Für ein solches Kontaktglas kann man zweckmäßigerweise den Randbereich als Fassung ausgestalten.

Je nach Fertigungsmethode und verwendeten Materialien können der zentrale Bereich und der Randbereich einstückig gebildet sein. Bei der Verwendung eines Kunststoffmaterials kommt dann beispielsweise ein Spritzgussverfahren für die Herstellung in Frage.

Um die gewünschte feste mechanische Kopplung zwischen Objekt und Laserbearbeitungsvorrichtung sicherzustellen, ist es ratsam, den Adapter nicht nur am Objekt anzulegen, sondern dort auch zu befestigen. Um den zentralen Bereich, der im Strahlengang der Laserbearbeitungsvorrichtung zu liegen kommt, in seiner optischen Funktion möglichst wenig zu beeinträchtigen, bietet es sich dazu an, nötige Fixierungsmittel zur Befestigung am Objekt am Randbereich vorzusehen. Die Fixierungsmittel, hier kommt beispielsweise im Falle augenchirurgischer Verfahren eine Saugkanalstruktur für Unterdruckbefestigung in Frage, bewirken dann, dass der zentrale Bereich im Kontakt mit dem Objekt liegt.

Die Erfindung wird nachfolgend unter Bezugnahme auf die Zeichnungen näher erläutert. In den Zeichnungen zeigt:
- Fig. 1: eine schematische Darstellung einer Laserbearbeitungsvorrichtung für ein augenchirurgisches Verfahren,
- Fig. 2: eine schematische Darstellung der Augenhornhaut eines Patienten,
- Fig. 3: eine Schnittdarstellung durch ein Kontaktglas für die Laserbearbeitungsvorrichtung der Fig. 1 mit einer schematischen Darstellung der Befestigung des Adapters,
- Fig. 4: den Adapter der Fig. 3 in einer Seitenansicht,
- Fig. 5: eine Schnittdarstellung durch einen andersartig ausgebildeten Adapter, der durch Unterdruck befestigt wird,
- Fig. 6: einen weiteren durch Unterdruck zu befestigenden Adapter und
- Fig. 7: eine Weiterbildung des Adapters der Fig. 5.

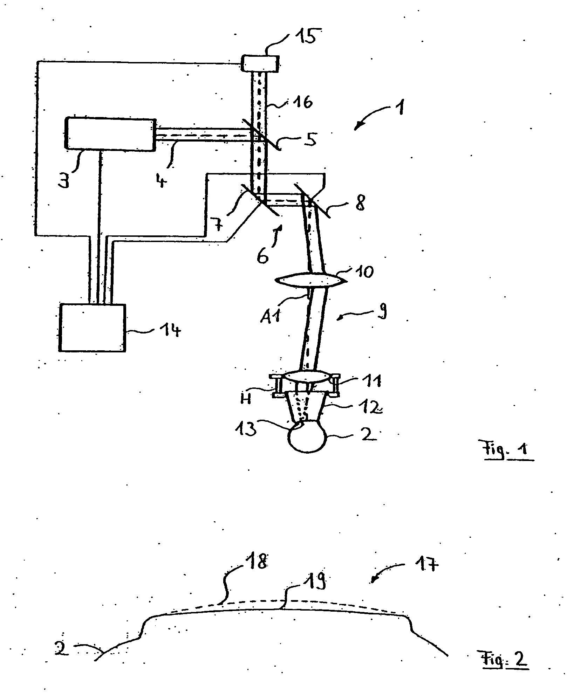

Fig. 1 zeigt ein Behandlungsgerät für ein augenchirurgisches Verfahren ähnlich dem in der EP 1159986 A1 bzw. der US 5549632 beschriebenen. Das Behandlungsgerät 1 der Figur 1 dient dazu, an einem Auge 2 eines Patienten eine Fehlsichtigkeitskorrektur gemäß dem bekannten LASIK-Verfahren auszuführen. Dazu weist das Behandlungsgerät 1 einen Laser 3 auf, der gepulste Laser-Strahlung abgibt. Die Pulsdauer liegt dabei z.B. im Femtosekundenbereich, und die Laserstrahlung wirkt mittels nichtlinearer optischer Effekte in der Hornhaut auf die eingangs beschriebene Art und Weise. Der vom Laser 3 entlang einer optischen Achse A1 abgegebene Behandlungsstrahl 4 fällt dabei auf einen Strahlteiler 5, der den Behandlungsstrahl 4 auf eine Scaneinrichtung 6 leitet. Die Scaneinrichtung 6 weist zwei Scanspiegel 7 und 8 auf, die um zueinander orthogonale Achsen drehbar sind, so dass die Scaneinrichtung 6 den Behandlungsstrahl 4 zweidimensional ablenkt. Eine verstellbare Projektionsoptik 9 fokussiert den Behandlungsstrahl 4 auf bzw. in das Auge 2. Die Projektionsoptik 9 weist dabei zwei Linsen 10 und 11 auf. Das Behandlungsgerät 1 stellt eine Laserbearbeitungsvorrichtung dar.

Der Linse 11 ist ein Adapter 12 nachgeordnet, der über eine Halterung H fest mit der Linse 11 1 und damit dem Strahlengang des Behandlungsgerätes 1 verbunden ist. Der noch näher zu beschreibende Adapter 12 liegt an der Hornhaut des Auges 2 an. Die optische Kombination aus Behandlungsgerät 1 mit daran befestigtem Adapter 12 bewirkt, dass der Behandlungsstrahl 4 in einem in der Hornhaut des Auges 2 gelegenen Fokus 13 gebündelt wird.

Die Scaneinrichtung 6 wird ebenso wie der Laser 3 und die Projektionsoptik 9 über (nicht näher bezeichnete) Steuerleitungen von einem Steuergerät 14 angesteuert. Das Steuergerät 14 bestimmt dabei die Lage des Fokus 13 sowohl quer zur optischen Achse A1 (durch die Scanspiegel 7 und 8) sowie in Richtung der optischen Achse A1 (durch die Projektionsoptik 9) vor.

Das Steuergerät 14 liest weiter einen Detektor 15 aus, der von der Hornhaut rückgestreute Strahlung, die den Strahlteiler 5 als Rückstrahlung 16 passiert, ausliest. Mittels des Detektors 15 kann der Betrieb des Lasers 3 sehr exakt gesteuert werden.

Der Adapter 12 sorgt dafür, dass die Hornhaut des Auges 2 eine gewünschte Soll-Form erhält. Das Auge 2 befindet sich aufgrund der Anlage der Hornhaut 17 am Adapter 12 in vorbestimmter Lage zum Adapter 12 und damit zum damit verbundenen Behandlungsgerät 1.

Dies ist schematisch in Fig. 2 dargestellt, die einen Schnitt durch die Augenhornhaut 17 zeigt. Um eine exakte Positionierung des Fokus 13 in der Augenhornhaut 17 zu erreichen, muss die Krümmung der Augenhornhaut 17 berücksichtigt werden. Die Augenhornhaut 17 weist eine Ist-Form 18 auf, die von Patient zu Patient unterschiedlich ist. Der Adapter 12 liegt nun an der Augenhornhaut 17 derart an, dass er diese in eine gewünschte Soll-Form 19 verformt. Der genaue Verlauf der Soll-Form 19 hängt dabei von der Krümmung der dem Auge 2 zugewandten Fläche des Adapters 12 ab. Durch den Adapter 12 sind bekannte geometrische und optische Verhältnisse für das Einbringen und Fokussieren des Behandlungsstrahls 4 in die Hornhaut 17 gegeben. Da die Hornhaut 17 am Adapter 12 anliegt und dieser wiederum über die Halterung H gegenüber dem Strahlengang des Behandlungsgerätes 1 ortsfest ist, kann der Fokus 13 durch Ansteuerung der Scaneinrichtung 6 sowie der verstellbaren Projektionsoptik 9 dreidimensional exakt in der Hornhaut 17 positioniert werden.

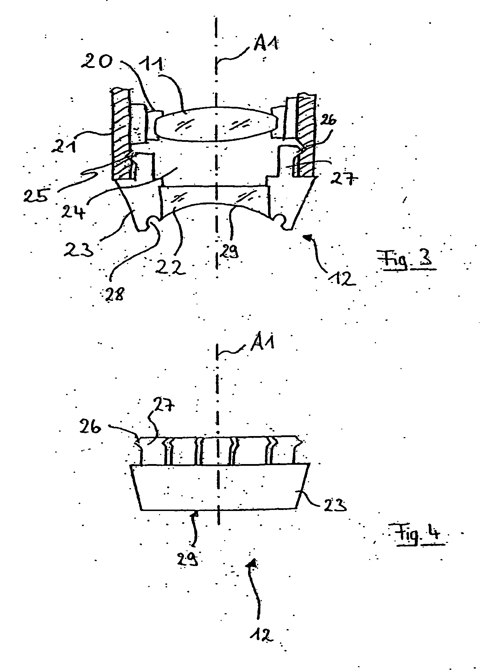

Fig. 3 zeigt die Halterung H des Adapters 12 im Detail. Wie zu sehen ist, befindet sich die vorderste Linse 11 des Behandlungsgerätes 1 in einer Linsenfassung 20, die wiederum in einer zylindrischen Aufnahme 21 sitzt.

Der Adapter 12 ist zweiteilig aufgebaut und besteht aus einem Kontaktglas 22, das in eine Fassung 23 geklebt ist. Durch Ansaugen der Fassung 23 auf die Augenhornhaut 17 wird das in die Fassung 23 geklebte Kontaktglas 22 mit seiner Unterseite 29 auf die Augenhornhaut 17 gepresst, so dass die zuvor bereits erläuterte gewünschte Soll-Form 19 gewährleistet ist.

Anstelle der zweiteiligen Ausführung gemäß Fig. 3 kann der Adapter 12 auch einstückig ausgebildet werden. Fassung 23 und Kontaktglas 22 sind dann aus einem durchgehenden Teil hergestellt, beispielsweise durch Spritzgussverfahren oder durch ein zerspanendes Verfahren aus einem einzigen Rohteil. Die hier beschriebenen Varianten des Adapters 12 können grundsätzlich mehrteilig oder auch einteilig realisiert werden, insbesondere was Kontaktglas 22 und Fassung 23 angeht.

Die Aufnahme 21 ist als Bohrung 24 ausgeführt, an deren Innenseite eine Ring-Nut 25 gebildet ist. Der Durchmesser der Bohrung 24 ist so bemessen, dass der zylinderförmige Ansatz an der Fassung 23 in die Bohrung 24 eingeschoben werden kann.

In die Nut 25 greifen Nasen 26 von Zungen 27 ein, die durch einen am behandlungsseitigen Ende der Fassung 23 vorgesehenen röhrenförmigen Ansatz gebildet sind. Wie die in Fig. 4 zu sehende Seitenansicht des Adapters 12 zeigt, sind die Zungen durch Trennschnitte im röhrenförmigen Ansatz gebildet. Die Zungen 27 sind dadurch flexibel und können beim Einschieben der Zungen in die Bohrung 24 nach innen gebogen werden.

Gelangen die Nasen 26 in die Nut 25, federn die Zungen 27, zurück und die Fassung 23 mit Kontaktglas 22 ist in der Aufnahme 21 gehalten.

Anstelle der Nut 25 kann auch ein geeigneter Rücksprung oder eine geeignete Erweiterung in der Aufnahme 21 vorgesehen werden.

Die axiale Lage, d.h. die Lage auf der optischen Achse A1 ist durch einen (in Fig. 3 nicht näher bezeichneten) als Anschlag dienenden Kragen, der an eine entsprechende Gegenfläche der Aufnahme 21 gezogen ist, definiert. Durch die Befestigung ist das Kontaktglas zur optischen Achse A1 ausgerichtet; gleiches gilt durch eine an der Unterseite 29 des Kontaktglases 22 mittels des Saugkanals 28 angedrückte Augenhornhaut 17.

Im befestigten Zustand haken die Nasen 21 in der Nut 25 ein. Die Fassung 23 mit Kontaktglases 22 ist somit fixiert und kann nicht mehr ohne zusätzlichen Kraftaufwand aus der Aufnahme 21 gezogen werden. Zum Entfernen muss deutlich mehr Kraft aufgewendet werden, als zum Einschieben. Dies kann durch geeignete Ausbildung eines Einführkegels an der von der Linse 11 abgewandten Vorderkante der Aufnahme 21 noch verstärkt werden. Die zum Herausziehen des Adapters 12 erforderliche Kraft kann durch die von der Linse 11 wegliegenden Seiten der Nasen 26 eingestellt werden. Im Ausführungsbeispiel der Fig. 3 sind dort Schrägflächen vorgesehen. Die Wahl des Winkels dieser Schrägflächen stellt die zum Herausziehen des Adapters 12 notwendige Kraft ein.

Möchte man eine große Mindestzugkraft zum Entnehmen des Adapters 12 aus der Aufnahme 21 erreichen, kann die Unterseite der Nasen 26 geeignet gestaltet werden, um einen Formschluss zu erreichen.

Zusätzlich kann man eine oder mehrere der Zungen 27 so ausbilden, dass sie beim Herausziehen der Fassung 23 aus der Aufnahme 21 abbrechen. Dies kann beispielsweise durch eine Sollbruchstelle an einer Zunge 27 erreicht werden. Durch diesen Ansatz kann wirkungsvoll vermieden werden, dass ein Kontaktglas 22 mit der Fassung 23 mehrfach verwendet wird, wenn eine Einweg-Verwendung vorgeschrieben ist, z.B. aus Gründen der Sterilität.

Das Konzept mit Zungen 27 und Nasen 26 kann natürlich auch derart invertiert werden, dass die Nasen 26 bezüglich der optischen Achse A1 innen liegen. Die Aufnahme 21 ist dann nicht als Bohrung 24 auszuführen, sondern als Außen-Kreiszylinder, dessen Nut 25 dann außen gebildet ist. Die Fassung 23 wird dann über die Aufnahme 21 geschoben.

Natürlich kann das Konzept mit Zunge und Aufnahme auch vertauscht werden, so dass die Zungen am Behandlungsgerät 1 und die Aufnahme an der Fassung 23 vorgesehen ist.

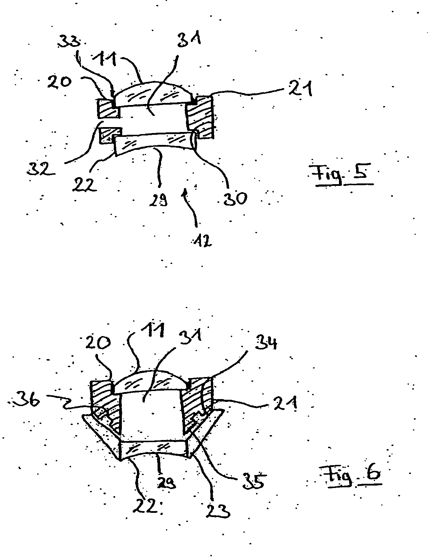

Fig. 5 zeigt eine andere Ausführungsform des Adapters 12. Hier wird der als fassungsloses Kontaktglas 22 gestaltete Adapter 12 in eine Aufnahme 21 eingeschoben, die an ihrem unteren Ende, d.h. der der Linse 11 abgewandten Seite, eine am Innenumfang umlaufende Stufe hat. Die Aufnahme 21 ist bei dieser Ausführungsform Teil der Linsenfassung 20, die sich röhrenförmig unterhalb der Linse 11 fortsetzt.

Dadurch ist ein Anschlag gebildet, bis zu dem das Kontaktglas 22 in den Adapter 12 eingeschoben werden kann. Das Kontaktglas 22 kommt so mit einem ringförmigen Randbereich an der in der Aufnahme 21 gebildeten Anschlagfläche 30 zu liegen. Kontaktglas 22, Aufnahme 21 sowie Linse 11 bilden einen Hohlraum 31, der über einen seitlichen Unterdruckanschluss 32 evakuiert werden kann. Zur Verbesserung der Dichtigkeit bzw. zur Minderung der erforderlichen Saugleistung ist die Linse 11 in ihre Fassung 20 zusätzlich mittels einer Dichtung, z.B. durch einen Dichtring, oder mittels einer Klebung 33 dichtend eingesetzt.

Das Kontaktglas 22 stützt sich somit durch Unterdruck gehalten mit seinem ringförmigen Randbereich an der Anschlagfläche 30 ab. Durch die Bohrung in der Aufnahme 21 ist dabei zugleich eine Zentrierung des Kontaktglases erreicht. Möchte man diese Zentrierung noch steigern, kann der Randbereich des Kontaktglases 22 kegelstumpfförmig ausgebildet werden und die Bohrung in der Aufnahme 20 erhält eine entsprechende sich verjüngende Kegelform.

Dieses Prinzip der Selbstzentrierung ist in der Bauweise der Fig. 6 in anderer Weise ausgenutzt. Das Kontaktglas 22 ist hier in einer Fassung 23 gehalten, die eine zum Behandlungsgerät 1 hin aufweitende konische Flanschfläche 34 hat. Die Aufnahme 21 weist eine entsprechende Gegenfläche als Anlagefläche 35 auf. In dieser kegeligen Anlagefläche 35 ist eine umlaufende Einfräsung 36 vorgesehen, die mit einer (nicht dargestellten) Vakuumleitung verbunden ist. Durch Unterdruck wird die Fassung 23 mit ihrer Flanschfläche 34 auf die Anlagefläche 35 gezogen und damit zugleich das Kontaktglas 22 zentriert.

Um einen besonders guten Unterdruckaufbau zu erreichen, ist es vorteilhaft, die Fassung 23 zumindest an der Flanschfläche 34 mit einem flexiblen Material, wie z.B. Silikon, zu versehen, da ein solches Material zu einem besonders guten Luftabschluss und damit zu einer guten Haltekraft führt. Diese Maßnahme kann natürlich auch bei der Ausführungsform der Fig. 5 eingesetzt werden.

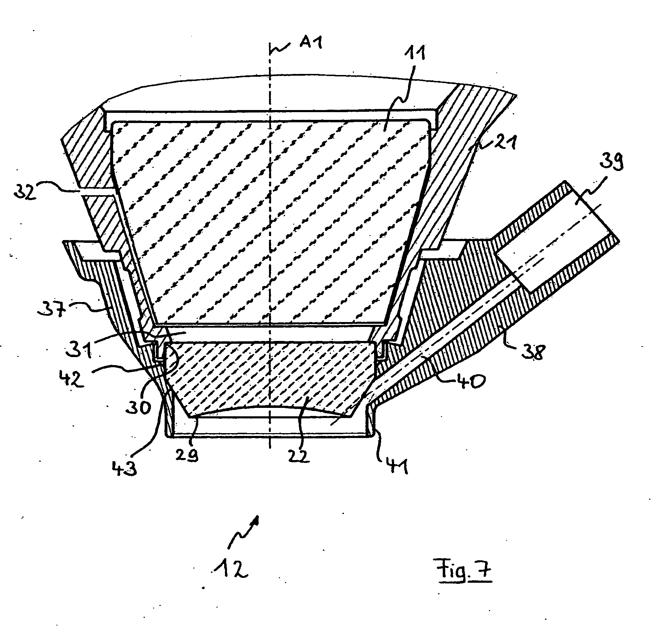

Fig. 7 zeigt einen Adapter 12, der das Befestigungsprinzip des Adapters der Fig. 5 aufgreift. Gleiche Elemente sind mit denselben Bezugszeichen versehen, so dass diesbezüglich auch auf die Beschreibung zu Fig. 5 verwiesen wird. In Abweichung zur Bauweise der Fig. 5 ist das Kontaktglas 22 allerdings mit einem als Fassung dienenden Kontaktglashalter 37 versehen.

Das Kontaktglas 22 sitzt im Kontaktglashalter 37. Es weist einen sich zum Auge hin verjüngenden konischen Abschnitt auf, der an einer konischen Ringfläche 43 des Kontaktglashalters 37 anliegt. Zur Befestigung des Kontaktglases 22 im Kontaktglashalter 37 kann an dieser Stelle eine Verklebung erfolgen. Alternativ oder zusätzlich ist eine Verklebung im Bereich des zylindrischen Kontaktglasabschnittes möglich. Dazu weist zweckmäßigerweise der Kontaktglashalter 37 in der Bauweise der Fig. 7 eine geeignete kegelförmige Abflachung auf, in die nach Einsetzen des Kontaktglases 22 in den Kontaktglashalter 27 Klebstoff eingebracht wird, so dass der Kontaktglashalter 37 an Kontaktglas 22 befestigt ist.

Der Kontaktglashalter 37 wirkt an der Befestigung des Kontaktglases 22 nicht mit, das wie bei der Bauweise der Fig. 5 auch mit einem ringförmigen Randbereich der oberen, d.h. der Laserbearbeitungsvorrichtung zugewandten Fläche mittels Unterdruck an die Anschlagfläche 30 gezogen wird. Der Unterdruck im Hohlraum 31 wird wiederum über einen Unterdruckanschluss 32 aufgebracht, der als Kanal durch die Aufnahme 21 des Behandlungsgerätes 1 ausgebildet ist. Der Kanal kann dabei in der die im Ausführungsbeispiel der Fig. 7 trichterförmig ausgebildete Aufnahme 21 ausgebildet werden. Auch ist es möglich, den Kanal durch eine entsprechende Nut in der Aufnahme 21 zu gestalten, so dass er durch die trichterförmige Aufnahme 21 und die eingesetzte vorderste Linse 11 begrenzt ist. Da die Linse 11 ihrerseits in der Aufnahme 21 dichtend befestigt, beispielsweise verklebt ist, führt ein Anlegen von Unterdruck am Unterdruckanschluss 32 zu einer Evakuierung des Hohlraumes 31, der das Kontaktglas 22 auf die Anschlagfläche 30 zieht. Ein Lösen dieser Unterdruckbefestigung gelingt mit einer Kraft, welche die von der Unterdruckbefestigung ausgeübte Saugkraft überwindet. Durch Einstellen der Saugleistung am Unterdruckanschluss 32 kann man die für die Panikverschlussfunktion Kraft, die zum Lösen des Kontaktglases 22 von der Anschlagfläche 30 nötig ist, wunschgemäß einstellen.

Die Mittel zur Zentrierung des Kontaktglases 22 sind hier beispielhaft als Passung ausgebildet, die von einem zylinderförmigen Kragen gebildet wird, der parallel zur optischen Achse A1 von der Anschlagfläche vorsteht und in den beim Befestigen das Kontaktglas 22 in axialer Richtung auf die Anschlagfläche 30 hin eingeführt wird.

Zur Befestigung des Kontaktglases 22 am Objekt, d.h. am Auge, weist der Kontaktglashalter 37 einen Stutzen 38 auf, der über einen Luer-Lock-Anschluss 39 sowie einen damit verbundenen Saugkanal 40 verfügt. Der Saugkanal 40 mündet seitlich am Kontaktglas 22 oberhalb eines Ansatzringes 41 des Kontaktglashalters 37, der gegenüber der Unterseite 29 des Kontaktglases 22 in axialer Richtung vorsteht. Dieser Ansatzring tritt beim Aufsetzen des Kontaktglases 22 zuerst in Kontakt mit der Augenhornhaut 17. Durch Applikation von Unterdruck am Luer-Lock-Anschluss 39 wird dann die Einheit aus Kontaktglashalter 37 und Kontaktglas 29 auf das Auge hin gezogen, so dass die Unterseite 29 des Kontaktglases wie bereits erläutert, die gewünschte Soll-Form 19 der Augenhornhaut 17 sicherstellt.

Da die Unterdruckverhältnisse beim Ansaugen des Kontaktglases 22 auf die Augenhornhaut 17 sehr viel stärker variieren können, als die Druckverhältnisse im Hohlraum 31 ist es zu bevorzugen, die Unterdruckbefestigung des Kontaktglases 22 mit Kontaktglashalter 37 am Auge sehr viel stärker auszulegen, als die Unterdruckbefestigung des Kontaktglases 22 an der Anschlagfläche 30. Bei einem panikbedingten Lösen des Auges vom Behandlungsgerät 1 geht dann mit einer sehr genau einstellbaren bzw. eingestellten Kraft die Verbindung zwischen Kontaktglas 22 und Aufnahme 21 auf; das Kontaktglas 22 mit Kontaktglashalter 37 verbleibt selbst am Auge.

## Patentansprüche

1. Kombination aus einer Laserbearbeitungsvorrichtung (1) und einem Adapter (12) zum mechanischen Koppeln eines Objektes (17) mit der Laserbearbeitungsvorrichtung (1), die zur Befestigung des Adapters (12) eine Aufnahme (21) bereitstellt, wobei
- der Adapter einen zentralen Bereich (22) aufweist, der in den auf einer optischen Achse (A1) zum Objekt (17) verlaufenden Strahlengang der Laserbearbeitungsvorrichtung (1) schaltbar ist, wobei der zentrale Bereich als Kontaktglas (22) zum Auflegen auf das Objekt (17) ausgebildet ist,
- der Adapter einen außerhalb des zentralen Bereichs (22) liegenden Randbereich aufweist, der beim Einsatz des Adapters (12) nicht im Strahlengang liegt,
- der Adapter (12) einerseits an der Laserbearbeitungsvorrichtung (1) befestigbar und andererseits mit seinem zentralen Bereich (22) zur Anlage am Objekt (17) ausgebildet ist,
- am Randbereich Befestigungsmittel vorgesehen sind, mit denen der Adapter (12) lösbar an der Aufnahme (21) der Laserbearbeitungsvorrichtung (1) so befestigbar ist, dass er mittels einer im wesentlichen entlang der optischen Achse verlaufenden Einsetzbewegung in die Aufnahme (21) einsetzbar und von dieser lösbar ist,
- die Befestigungsmittel eine Flanschfläche (34) zur Befestigung mittels Unterdruck oder elektrostatischer oder elektromagnetischer Effekte aufweisen und
- der Randbereich des Adapters (12) und die Aufnahme (21) so ausgebildet sind, dass sie zusammen eine zentrierende Befestigung des Adapters (12) an der Laserbearbeitungsvorrichtung (1) bewirken,
-- wobei der Randbereich des Adapters (12) kegelstumpfförmig ausgebildet ist und die Aufnahme (21) eine Bohrung aufweist, die passend kegelförmig ist, so dass der kegelstumpfförmige Randbereich des Adapters (12) vom Unterdruck oder den elektrostatischen oder elektromagnetischen Effekten in die sich verjüngende Bohrung gezogen wird,
-- oder wobei der Randbereich des Adapters (12) sich kegelförmig zur Laserbearbeitungsvorrichtung hin aufweitet und die Aufnahme (21) dazu passend kegelstumpfförmig ist, so dass der Adapter (12) mittels Unterdruck oder den elektrostatischen oder elektromagnetischen Effekten zentrierend an der Laserbearbeitungsvorrichtung (1) befestigt wird.

2. Kombination nach Anspruch 1, **dadurch gekennzeichnet, dass** die Befestigungsmittel die Befestigung mittels Unterdruck bewirken und die Flanschfläche (34) an einer Fassung (23) des zentralen Bereiches ausgebildet ist.

3. Kombination nach Anspruch 1, **dadurch gekennzeichnet, dass** die Befestigungsmittel die Befestigung mittels Unterdruck bewirken und die Flanschfläche als den zentralen Bereich (22) umgebender ringförmiger Randbereich ausgebildet ist und dass der zentrale Bereich (22) dazu ausgebildet ist, mit der Aufnahme (21) und einer dort dichtend eingesetzten Linse (11) einen zu der Befestigung mittels Unterdruck dienenden Hohlraum (31) zu bilden.

4. Kombination nach Anspruch 1, **dadurch gekennzeichnet, dass** der Randbereich und der zentrale Bereich (22) einstückig ausgebildet sind.

5. Kombination nach Anspruch 1, **dadurch gekennzeichnet, dass** der Randbereich als Fassung (23) des Kontaktglases (22) ausgebildet ist.

6. Kombination nach einem der obigen Ansprüche, **dadurch gekennzeichnet, dass** am Randbereich (23) Fixierungsmittel (28, 41, 40) vorgesehen sind, mit denen der zentrale Bereich (22) im Kontakt mit dem Objekt (17) haltbar ist.

7. Kombination nach Anspruch 6, **dadurch gekennzeichnet, dass** die Fixierungsmittel (28, 41, 40) für eine Unterdruckbefestigung ausgebildet sind.

## Claims

1. Combination of a laser processing device (1) and an adapter (12) for mechanically coupling an object (17) to the laser processing device (1), which provides a receptacle (21) for fastening the adapter (12), wherein
- the adapter comprises a central region (22) which can be moved into the beam path of the laser processing device (1) extending along an optical axis (A1) up to the object (17), wherein the central region is formed as a contact glass (22) to be placed on the object (17),
- the adapter has an edge region lying outside the central region (22), which edge region does not lie in the beam path when the adapter (12) is used,
- the adapter (12) on the one hand can be fastened to the laser processing device (1) and on the other hand is formed with its central region (22) for mounting on the object (17),
- fastening means are provided on the edge region by means of which the adapter (12) can be detachably fastened to the receptacle (21) of the laser processing device (1) so that it can be inserted into the receptacle (21) by means of an insertion movement extending substantially along the optical axis and can be detached from the receptacle,
- the fastening means comprise a flange surface (34) for fastening by means of a vacuum or electrostatic or electromagnetic effects, and
- the edge region of the adapter (12) and the receptacle (21) are formed so that together they effect a centring fastening of the adapter (12) on the laser processing device (1),
- wherein the edge region of the adapter (12) is formed having a frusto-conical shape and the receptacle (21) has a bore with a matching conical shape so that the frusto-conical edge region of the adapter (12) is drawn by a vacuum or the electrostatic or electromagnetic effects into the tapering bore,
- or wherein the edge region of the adapter (12) widens out conically to the laser processing device and the receptacle (21) has a matching frusto-conical shape so that the adapter (12) is fastened by means of a vacuum or the electrostatic or electromagnetic effects in a centring manner on the laser processing device (1).

2. Combination according to claim 1, **characterised in that** the fastening means effect the fastening by means of a vacuum and the flange area (34) is formed on a frame (23) of the central region.

3. Combination according to claim 1, **characterised in that** the fastening means effect the fastening by means of a vacuum and the flange area is formed as an annular edge region surrounding the central region (22) and that the central region (22) is formed so that together with the receptacle (21) and a lens (11) tightly inserted therein latter it forms a cavity (31) serving for the fastening by means of a vacuum.

4. Combination according to claim 1, **characterised in that** the edge region and the central region (22) are formed integrally.

5. Combination according to claim 1, **characterised in that** the edge region is formed as a frame (23) of the contact glass (22).

6. Combination according to one of the preceding claims, **characterised in that** fastening means (28, 41, 40) are provided on the edge region (23), by means of which the central region (22) can be held in contact with the object (17).

7. Combination according to claim 6, **characterised in that** the fastening means (28, 41, 40) are formed for fastening under a vacuum.

## Revendications

1. Combinaison constituée d'un dispositif d'usinage laser (1) et d'un adaptateur (12) pour le couplage mécanique d'un objet (17) avec le dispositif d'usinage laser (1), qui met à disposition un logement (21) pour la fixation de l'adaptateur (12),
- l'adaptateur comprenant une zone centrale (22), qui peut être branchée dans le trajet des faisceaux, s'étendant sur un axe optique (A1) en direction de l'objet (17), du dispositif d'usinage laser (1), la zone centrale étant conçue comme un verre de contact (22) destiné à être posé sur l'objet (17),
- l'adaptateur comprenant une zone de bord se trouvant à l'extérieur de la zone centrale (22), qui ne se trouve pas dans le trajet des faisceaux lors de l'insertion de l'adaptateur (12),
- l'adaptateur (12) pouvant d'une part être fixé au dispositif d'usinage laser (1) et d'autre part conçu avec sa zone centrale (22) destinée à être appuyée contre l'objet (17),
- des moyens de fixation étant prévus sur la zone de bord, avec lesquels l'adaptateur (12) peut être fixé de manière amovible au logement (21) du dispositif d'usinage laser (1) de façon à ce qu'il puisse être inséré dans le logement (21) et retiré de celui-ci à l'aide d'un mouvement d'insertion s'étendant essentiellement le long de l'axe optique,
- les moyens de fixation comprenant une surface de bride (34) pour la fixation, à l'aide d'une dépression ou d'effets électrostatiques ou électromagnétiques et
- la zone de bord de l'adaptateur (12) et le logement (21) sont conçus de façon à permettre ensemble une fixation centrée de l'adaptateur (12) sur le dispositif d'usinage laser (1),
- la zone de bord de l'adaptateur (12) étant conçu sous une forme tronconique et le logement (21) comprenant un alésage qui est de forme conique complémentaire, de façon à ce que la zone de bord tronconique de l'adaptateur (12) puisse être tirée par la dépression ou les effets électrostatiques ou électromagnétiques dans l'alésage se rétrécissant,
- ou la zone de bord de l'adaptateur (12) s'élargissant de manière conique en direction du dispositif d'usinage laser et le logement (21) étant de forme tronconique complémentaire, de façon à ce que l'adaptateur soit fixé de manière centrée à l'aide d'une dépression ou des effets électrostatiques ou électromagnétiques sur le dispositif d'usinage laser (1).

2. Combinaison selon la revendication 1, **caractérisée en ce que** les moyens de fixation permettent la fixation à l'aide d'une dépression et la surface de la bride (34) est conçue sur une monture (23) de la zone centrale.

3. Combinaison selon la revendication 1, **caractérisée en ce que** les moyens de fixation permettent la fixation à l'aide d'une dépression et la surface de bride est conçue comme la zone de bord annulaire entourant la zone centrale (22) et **en ce que** la zone centrale (22) est conçue pour former, avec le logement (21) et une lentille (11) insérée de manière étanche, un espace creux (31) servant de fixation à l'aide d'une dépression.

4. Combinaison selon la revendication 1, **caractérisée en ce que** la zone de bord et la zone centrale (22) sont formées d'une seule pièce.

5. Combinaison selon la revendication 1, **caractérisée en ce que** la zone de bord est conçue comme une monture (23) du verre de contact (22).

6. Combinaison selon l'une des revendications précédentes, **caractérisée en ce que**, sur la zone de bord (23) sont prévus des moyens de fixation (28, 41, 40), avec lesquels la zone centrale (22) peut être maintenue en contact avec l'objet (17).

7. Combinaison selon la revendication 6, **caractérisée en ce que** les moyens de fixation (28, 41, 40) sont conçus pour une fixation par dépression.
